# EUROPEAN PATENT APPLICATION

(11) **EP 0 665 290 A2**
(43) Date of publication of application: **02.08.1995**
(21) Application number: 95300555.0
(22) Date of filing: 30.01.1995
(51) Int. Cl.: C12N 15/00, C12N 5/04, A01H 5/00

(54) **Process for gene transfer into plant cells**

(30) Priority: 31.01.1994 JP 25956/94
(71) Applicant: DIRECTOR GENERAL OF NATIONAL AGRICULTURE RESEARCH CENTER, MINISTRY OF AGRICULTURE, FORESTRY AND FISHERIES, Tsukuba-shi, Ibaraki 305 (JP)
(72) Inventor: Otsuki, Yoshiaki, Tsukuba-shi, Ibaraki 305 (JP); Tsugawa, Hidehito, Tsukuba-shi, Ibaraki 305 (JP)
(74) Representative: Woods, Geoffrey Corlett

(57) **Abstract**

The present invention relates to a process for gene transfer into plant cells comprising treating the protoplasts of said cells with a DNA in the presence of a polycation to facilitate the intake of said DNA into said cells, a process for transforming a plant according to said process for gene transfer, and a transformed plant obtained according to said process for transforming.

Different from physical methods such as the electroporation technique and particle gun technique, there is provided a chemical method for gene transfer utilizing the ionic bonds among particles as well as the "substance-intake" action of protoplasts.

## Description

### BACKGROUND OF THE INVENTION

### 1. Field of the Invention

The present invention relates to a process for gene transfer into plant cells by utilizing the action of a chemical substance which bonds DNA with plant protoplasts.

### 2. Description of the Prior Art

As biotechnology has advanced, a possibility of growing useful plant varieties by means of gene transfer has increased.

With respect to the techniques for gene transfer into plant cells, there have already been developed the Agrobacterium mediated gene transfer, electroporation technique and particle gun technique. In the transformation of bacteria, the chemical method using calcium phosphate has also become a prevailing technique.

### OBJECTS AND SUMMARY OF THE INVENTION

The object of the present invention is to add, as a gene transfer technique for plants, a process for gene transfer into plant cells by utilizing the action of a chemical substance which bonds DNA with plant protoplasts.

The process for gene transfer into plant cells according to the present invention is characterized by treating plant protoplasts with a DNA in the presence of a polycation to thereby facilitate the intake of the DNA into the plant cells.

The process of the present invention utilizes the ionic bonds among particles as well as the "substance-intake" action of protoplasts. Since DNA and protoplasts respectively have a negative charge, DNA-intake into cells are facilitated by adding a polycation having a positive charge to thereby mediate the bonding of the DNA with the protoplasts.

In the present invention, a polycation means a high molecular ion having a positive charge along with its high molecular chain.

A polycation to be used in the present invention preferably has a molecular weight of from 100,000 to 300,000.

Preferably, a polycation to be used in the present invention is a polymer of a basic amino acid.

As to the polymer of a basic amino acid, either a homopolymer or a copolymer may be used, and any of the types of L-type, D-type and DL-type may be used.

Examples of homopolymers of a basic amino acid include polyornithine, polylysine and polyarginine. Among all, poly-L-ornithine is preferable.

With respect to the target plants of the present invention, there is no special limitation. Any plant may be used as long as the protoplast culture system therefor has been established.

A preferred embodiment of the present invention will be described taking an example where a foreign gene is transferred into Rice protoplasts by using polyornithine as a polycation.

In the process mentioned below, a DNA and a polycation are mixed into purified protoplasts to thereby transfer a foreign gene thereinto, and then the resultant protoplasts are cultured to express the transferred gene.

### (Purification of Protoplasts)

First, protoplasts are purified.

Cultured cells of Rice are treated with an enzyme. A protoplast solution which has been filtered through a 30 mesh filter is placed in a centrifuge tube, and centrifuged to thereby precipitate protoplasts. After the supernatant is removed, the tube is shaken to thereby loosen the protoplasts, and then a rinsing solution is added thereto to re-suspend the protoplasts. After the protoplasts are washed twice, they are suspended in a small amount of mannitol solution, and then the concentration of cells is determined. Preferably, a mannitol solution of 0.5-0.7 M is used. (This is the same for any mannitol solution to be used in the present invention.)

### (Preparation of Protoplast Suspension)

The cell concentration is determined by using a hemocytometer. The protoplast concentration has been arranged to give approximately 3x10⁷ protoplasts/ml.

### (Stock Solutions)

The kinds of stock solutions to be used and the concentrations thereof at use are shown in Table 1.

**Table 1**

| Stock solution | Concentration at use |
|---|---|
| Phosphate buffer (0.5 M) | 0.025 M |
| Polyornithine (1 mg/ml) | 4 µg/ml |
| DNA (2 mg/ml) | 2 µg/ml |
| Mannitol | 0.5-0.7 M |
| Protoplast | (1.5x10⁶ /ml) |

As to the phosphate buffer, preferably a potassium salt buffer is used. As to the polyornithine, preferably one with a molecular weight of from 100,000 to 300,000 is used. As to the DNA to be transferred, preferably a DNA which has been incorporated in an expression vector and purified is used.

### (Mixing Procedures)

It is preferred that the mixing procedures be carried out as follows.

### 1. Dilution of Stock Solutions

First, the stock solutions of polyornithine and DNA are respectively diluted with a mannitol solution up to the 10-fold concentrations of their concentrations at use. In other words, the concentration of the polyornithine solution is made 40 µg/ml, and that of the DNA solution 20 µg/ml. During this process, preferably these solutions are respectively agitated well to a homogeneous concentration with a vortex mixer.

### 2. Preparation of Inoculation Solution

First, a mannitol solution is placed in a test tube, to which a phosphate buffer (0.5 M), a polyornithine solution (40 µg/ml) and a DNA solution (20 µg/ml) are added in this order. Then, the tube is agitated. After that, the tube is stood in ice to cool for about 10 minutes. During this period, the polyornithine and DNA form a complex, which then waits for the addition of protoplasts.

In this process, if the final volume of solution per one test tube is 5 ml, the preferable amount of each solution to be used is 1.25 ml for the mannitol solution, 0.25 ml for the phosphate buffer (0. 5 M), 0.5 ml for the polyornithine solution (40 µg/ml) and 0.5 ml for the DNA solution (20 µg/ml).

### 3. Mixing of the Inoculation Solution and Protoplast Solution

Next, an equivalent volume of the protoplast solution is mixed to the inoculation solution. In this process, it is preferred that the surface of the protoplasts be kept clean by precipitating the protoplasts immediately before the addition to the inoculation solution by centrifugation, re-suspending them in a fresh mannitol solution and then adding them to the inoculation solution. Preferably, this operation is carried out while the test tube containing the inoculation solution is stood in ice.

The number of the protoplasts to be added to the inoculation solution will be 7.5x10⁶ per a test tube. Therefore, it is preferred that a certain amount of the protoplast stock solution (of which the concentration has been determined) is poured into each of thin test tubes wherein about 3 ml of a mannitol solution has been put in advance. The protoplasts are precipitated by centrifugation and the supernatant is removed. This removal should be carried out in a manner that no precipitation is whirled up.

One of the above-mentioned thin test tube is taken, and the precipitation therein is loosened. Then, 2.5 ml of a cooled mannitol solution is added thereto, and the resultant mixture is added to the test tube containing the inoculation solution.

The test tube is allowed to stand in ice for about 10 minutes. During this period, the complex of DNA and polyornithine is taken into the protoplasts.

### (Centrifugation/Removal of Supernatant/Addition of Medium)

After the inoculation, the protoplasts are precipitated by centrifugation and washed once with a mannitol solution. Then, a small amount of medium for protoplasts is added thereto.

### (Preparation of Medium for Protoplasts)

For the culture of the protoplasts, a liquid medium generally called a conditioning medium, or a mixture of equal amounts of the conditioning medium and a medium for protoplasts is used. The "conditioning medium" used in the present invention means the medium which is obtained by culturing cells of Rice or the like on a medium for protoplasts and removing the cultured cells therefrom.

### (Pouring into Eppendorf Tubes)

In an experiment of the "transient assay" to detect the expression of a gene, protoplasts are placed in Eppendorf tubes, which are then laid down during their culture. If the transferred gene is β - glucuronidase (GUS) gene, GUS activity can be sufficiently detected in one day culture.

In order to obtain a transformed plant, the cells into which a foreign gene has been transferred are cultured while promoting the early division of the protoplasts according to the culture method using the above-mentioned conditioning medium or the nurse culture method.

A part of the foreign gene which has been transferred into the cells according to the process of gene transfer of the present invention is incorporated in the nuclear DNA, and proliferates with cell division. Thus, a transformed plant is obtained.

Different from the physical methods such as the electroporation technique and particle gun technique, the process for gene transfer of the present invention is a chemical method utilizing the ionic bonds among particles as well as the "substance-intake" action of protoplasts.

According to the process for gene transfer of the present invention, the damage to protoplasts is small. Furthermore, neither a carrier DNA nor expensive machinery is necessary. In addition, a large quantity of protoplasts can be treated.

### BRIEF DESCRIPTION OF THE DRAWINGS

Fig. 1 is a graph showing the influence of the polycation concentration in the process of the present invention.

Fig. 2 is a photograph showing morphologies of an organism exhibiting colony formation on the hygromycin selective medium. EP means the electroporation technique, and PLO the process of the present invention using poly-L-ornithine.

### DESCRIPTION OF THE PREFERRED EMBODIMENTS

Now the present invention will be described in more detail with reference to Examples, which should not be construed to be limiting the scope of the present invention.

### EXAMPLE 1

### Gene Transfer Efficiency Examined by the Transient Assay System

Protoplasts were prepared from suspension-cultured cells of a Rice variety "Nipponbare" according to conventional methods.

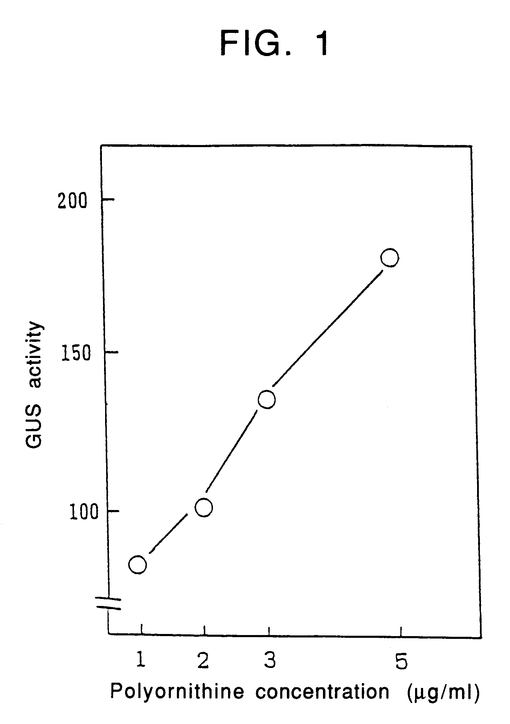

Into 0.5 M mannitol solution, potassium phosphate buffer (pH 5.6) and 35S-GUS were added at the final concentration of 0.025 M and 2 µg/ml, respectively. Poly-L-ornithine (molecular weight: about 145,000) was added thereto at varied concentrations of 1-5 µg/ml (final concentration), and the resultant mixtures were left standing for about 10 minutes. To each of these mixtures, an equal volume of protoplast suspension (3x10⁶/ml) was added and left standing for about 10 minutes. Then, the protoplasts were harvested by low rate centrifugation. The inoculated protoplasts were cultured for one day, and then the expression of GUS gene was quantitated with a fluorescent spectrophotometer. The results are shown in Fig. 1.

The GUS activity increased as the polycation concentration increased. From this, it is has been found that the gene transfer efficiency is closely related with the concentration ratio of the DNA and polycation.

### EXAMPLE 2

### Acquisition of a Resistant Callus by the Transfer of Hygromycin Resistant Gene

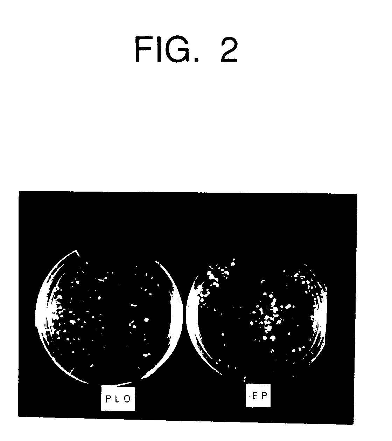

Into 0.5 M mannitol solution, potassium phosphate buffer (pH 5.6) and hygromycin resistant gene were added at the final concentration of 0.025 M and 2 µg/ml, respectively. 1 µg/ml (final concentration) of poly-L-ornithine (molecular weight: about 145,000) was added thereto, and the resultant mixture was left standing for about 10 minutes. To this mixture, an equal volume of protoplast suspension (3x10⁶/ml) was added and left standing for about 10 minutes. Then, the protoplasts were harvested by low rate centrifugation. The protoplasts obtained were cultured on the conditioning medium for 10 days, and then cultured on a medium containing 30 µg/ml of hygromycin B for one month. For the purpose of comparison, gene transfer by the electroporation technique was simultaneously carried out, and the formation of colonies was observed for both cases. Fig. 2 shows the formation of resistant colonies.

The transformation efficiency of the process of the present invention was almost equal to that of electroporation technique. From this result, it has been demonstrated that the process of the present invention is useful in the transformation of plants.

## Claims

1. A process for transferring a gene into a plant cell, which comprises treating the protoplast of said cell with a DNA encoding said gene in the presence of a polycation to thereby facilitate the intake of said DNA into said cell.

2. A process according to claim 1, wherein said polycation has a molecular weight of from 100,000 to 300,000.

3. A process according to claim 1 or claim 2, wherein said polycation is a polymer of a basic amino acid.

4. A process according to claim 3, wherein said polymer of a basic amino acid is polyornithine.

5. A process according to claim 3, wherein said polymer of a basic amino acid is poly-L-ornithine.

6. A process for obtaining a transformed plant, which comprises culturing a cell into which a foreign gene has been transferred according to the process of any one of claims 1 to 5.

7. A transformed plant which has been obtained according to the process of claim 6.

8. Use of a polycation for transferring a gene into plant cells.

9. Use according to claim 8 wherein the polycation is as defined in any one of claims 2 to 5.
